# EUROPEAN PATENT APPLICATION

(11) **EP 1 695 729 A1**
(43) Date of publication of application: **30.08.2006**
(21) Application number: 05251138.3
(22) Date of filing: 25.02.2005
(51) Int. Cl.: A61M 16/00, A61M 11/06, B65D 33/00, A61K 9/00

(54) **Medicament delivery device comprising a flexible bag**

(71) Applicant: Omron Healthcare Co., Ltd., Kyoto 615-0084 (JP)
(72) Inventor: Gee-Turner, Adrian, Omron Healthcare (UK) Ltd., Milton Keynes, Buckinghamshire, MK150DG (GB)
(74) Representative: Calderbank, Thomas Roger

(57) **Abstract**

This invention relates to devices, uses and methods for the delivery of a medicament in aerosol form. The proposed arrangement permits simple, cost-effective delivery (e.g. for mass immunisation programmes) which avoids contamination risk and/or disposal difficulties.

Medicament in aerosol form is supplied to the interior (12) of a bag (14), which is placed over a patient's (10) nose and mouth to enable inhalation of the medicament. A nebuliser (16) is used to generate the aerosol using a flow of compressed air, e.g. from a liquid formed from diluted medicament powder. The bag (14) has a constricted neck (32) for gripping, and a suitably sized opening (30) for fitting over the patient's nose and mouth. The bag may be edible, e.g. made of rice paper or food-based polymer.

## Description

This invention relates to devices, uses and methods for the delivery of a medicament in aerosol form.

A number of medicaments are suitable for delivery to the lungs in the form of an aerosol. These include certain vaccines such as measles vaccine. Delivery of medicaments in the form of an aerosol has a number of advantages over other routes of administration. In the case of vaccination, aerosol delivery to the lungs avoids many disadvantages associated with conventional injection delivery, such as cost of needles, safe disposal of used needles and fear of injections, especially among children. These disadvantages are particularly problematic for large-scale immunization programmes in economically underdeveloped countries. Moreover, medical trials suggest that the use of aerosols result in a higher immunisation coverage and fewer side effects than subcutaneous injection. Delivery of medicaments in aerosol form is also preferred for many pulmonary conditions such as asthma.

Conventional delivery systems for medicaments in aerosol form generally comprise means to convert a liquid medicament into an aerosol, such as a nebuliser, together with means to direct the aerosol into a patient's nose or mouth such as a mask or a rigid container with a nozzle. The patient inhales the aerosol from the mask or chamber. Conventionally, the mask or chamber is re-usable and often has one or more valves. Due to the risk of contamination, re-use of such systems requires frequent, thorough cleaning. Moreover, the complexity of the construction leads to considerable cost. Particularly in the setting of a major immunisation programme, cost, disposal difficulties and likelihood of contamination constitute substantial disadvantages.

The present invention seeks to provide arrangements for delivery of a medicament in aerosol form which are sufficiently simple and cost-effective to be used in a mass immunisation programme.

At its most general, the present invention proposes that the medicament is supplied in aerosol form to the interior of a bag of flexible material, and the bag is then placed over the nose and/or mouth of the person to be immunised (hereinafter "a patient"). The patient then inhales the air, and hence the aerosol, from the bag thereby delivering the medicament to the patient.

In such an arrangement, the bag will need to have an opening of suitable size to fit over the nose and/or mouth of the patient, and this can easily be achieved by suitable shaping of the opening of the bag. The aerosol of medicament is introduced into the bag from e.g. a nebuliser, and the opening of the bag is then closed to hold the aerosol within the bag. Provided that the time between the supply of aerosol into the bag and the time the bag is placed over the nose and/or mouth of the patient is reasonably short, manual constriction of the opening of the bag should provide sufficient sealing for there to be enough medicament in the bag when it is placed over the nose and/or mouth of the patient for the patient to inhale a suitable dose. Inhalation from and exhalation into the bag may be repeated to ensure that the patient inhales a suitably large dose.

The present invention has several aspects. Thus, the present invention may provide a kit of parts for use in delivery of a medicament to a patient, comprising:
at least one bag of flexible material;
a container for said medicament; and
means for generating an aerosol of at least part of said medicament in said container for supply of said aerosol to the interior of the bag

According to the second aspect of the present invention there may be provided a medicament delivery device comprising a bag of flexible material, and a medicament in aerosol form in said bag, wherein said bag is closed but is openable to fit over the nose and/or mouth of a patient.

Preferably, the bag is of edible material. This means that it can be ingested readily by the patient, which provides a means of disposal of the bag and so prevents cross-contamination from one patient to another. To achieve such an edible bag, the bag may be made of rice paper or a food-derived polymer or cellulose, vegetable or fruit. Moreover, it is preferable that the bag is impregnated with vitamins and/or nutritional minerals, so that those vitamins and/or nutritional minerals may be absorbed by the patient when the bag is ingested. This permits the bag to serve a second purpose after the medicament has been ingested.

A further possibility is for the bag to be at least partially dissolved in water or other liquid to soften it prior to being eaten. Softening may tend to cause vitamins and/or nutritional minerals in the bag to dissolve in the water, so that the water can then be drunk.

However, the present invention is not limited to the use of edible material. The use of paper, even if not readily edible, is preferred because of cost considerations. However, plastics materials may be used although they then introduce the issue of disposal.

According to a third aspect of the present invention there may be provided a bag for use in a method of delivery of a medicament in aerosol form to a patient, the bag being of edible material and having an opening for fitting over the nose and/or mouth of a patient.

According to a fourth aspect of the present invention there may be provided a bag for the production of a medicament delivery device for treatment of a vaccinable disease by supply of said medicament in aerosol form for inhalation by a patient the bag being of edible material and having an opening for fitting over the nose and/or mouth of a patient.

According to a fifth aspect of the present invention there may be provided a method of treatment of a patient, comprising:
introducing medicament in aerosol form into the hollow interior of a bag of flexible material, the bag having an opening for fitting over the nose and/or mouth of the patient through which opening the medicament is introduced into the bag;
temporarily closing the bag to retain the aerosol therein;
opening the bag and fitting the opening over the nose and/or mouth of the patient;
supplying the medicament to the patient by inhalation of the aerosol by the patient from the bag.

As mentioned above, the bag may be closed by manual constriction between the time in which the aerosol is supplied to the interior of the bag and the time at which the bag is placed over the nose and/or mouth of the user. To facilitate such manual constriction of the bag, the bag may have a neck of reduced width compared to the opening, so that the bag is splayed between the neck and the opening. In this way, the bag is closed by constriction at the neck to prevent loss of a significant amount of the aerosol, but the splaying of the bag enables it to be placed over the nose and/or mouth of the patient whilst the manual constriction is still present, so that there is no great loss of aerosol on release of the constriction. The neck also facilitates manual constriction of the bag onto e.g. a nozzle of the nebuliser which will supply the medicament in aerosol form.

It is convenient if the bag is supplied in the form of a sheet, e.g. of two-ply material with the plies sealed together to define the periphery of the bag. The sheet may be perforated in a shape conforming to the periphery of the bag so that a plurality of such sheets may be provided to a site where treatment of a plurality of patients is to be carried out, with the sheets being in a pad or block. Then, for each patient, a sheet is removed from the pad or block, perforations are broken to release the bag from the rest of the sheet, then medicament in aerosol form is supplied to the interior of the bag.

Another alternative is for such sheets or the bags themselves to be joined end-to-end and formed e.g. into a roll on a bobbin for storage and/or distribution. By perforating the joins between the bags/sheets it becomes possible to detach one bag/sheet from the roll, keeping the next bag/sheet clean until it is needed.

The present invention has been developed for the treatment of patients with a measles vaccine. However, the present invention is not limited to such vaccines, and the present invention may be used for other medicaments which can be supplied in aerosol form for use in an immunisation programme. The low cost nature of the present invention makes it particularly suitable for immunisation programmes in less developed countries.

Embodiments of the present invention will now be described in detail, by way of example, with reference to the accompanying drawings, in which:
Fig.1 is a general schematic view of the delivery of a medicament in accordance with the present invention;
Fig.2 illustrates the bag used in the present invention as part of a sheet;
Fig.3 illustrates a block of such sheets;
Fig.4 illustrates a roll of such sheets on a bobbin dispenser;
Fig. 5 illustrates a modification to the delivery arrangement of Fig. 1.

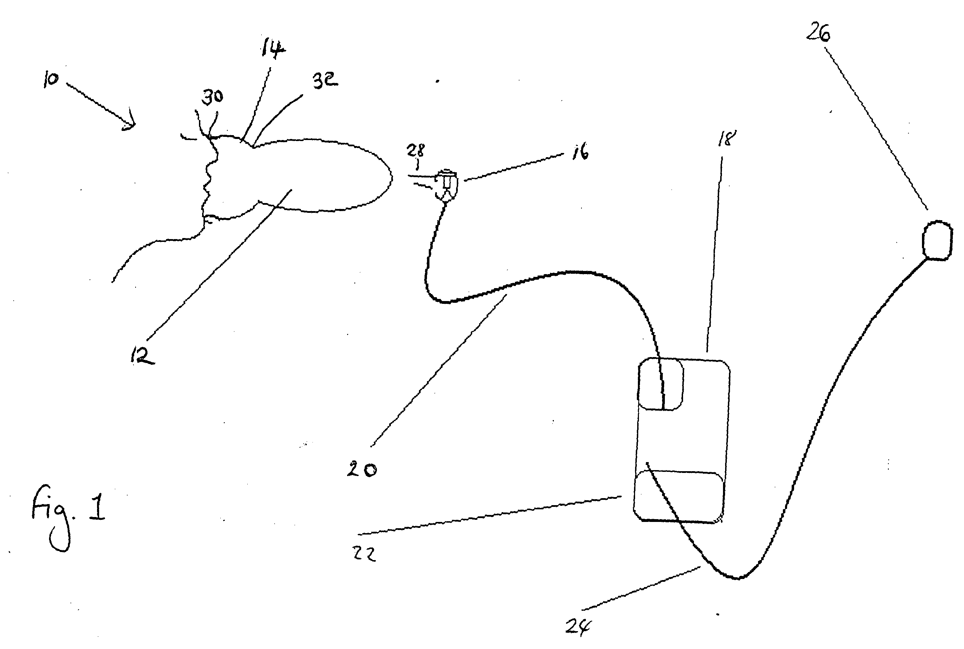

Referring first to Fig.1, a patient 10, is treated with a medicament, such as a measles vaccine, by inhaling the medicament in aerosol form from the interior 12 of a bag 14 of edible material. As illustrated in Fig.1, the bag 14 is placed over the mouth and nose of the patient 10, so that the patient 10 breathes the air, and hence the aerosol in the air, from the interior of the bag 14, thereby ingesting the aerosol.

Such a delivery arrangement is of particular value when the patient is a child or infant, since they will readily be able to breathe the air and aerosol in the bag 14 but will not be frightened or distressed by having the bag 14 placed over their nose and mouth. Moreover, the bag 14 may then be disposed of, rather than being re-used with another patient to prevent cross-contamination.

As mentioned above, the bag 14 is of edible material and it is envisaged that ingestion of the bag 14 itself by the patient represents the primary way of disposal of the bag 14. The bag 14 may thus be made of rice paper, which is easily digestible, or may be made of a polymer of food-based material. The bag 14 may be eaten directly, or may be immersed in water, at least partially to dissolve it, prior to ingestion.

Since the bag 14 is intended to be ingested, it is preferably impregnated with vitamins and/or nutritional minerals, so that those vitamins and/or minerals will be absorbed by the patient. Thus the patient not only receives a dose of the medicament, but also a dietary supplement. A flavour can be imparted to the bag as a reward and encouragement to the patient to eat the bag.

In this embodiment, a nebuliser 16 is used to fill the bag. Alternative means of generating an aerosol may be used, e.g. a spray of compressed gas having the medicament mixed therein. The nebuliser 16 is powered by a compressor 18 producing a flow of compressed air to the nebuliser 16 via a low pressure compressed air line 20. The compressor 18 may have a battery 22 and/or a power line 24 terminating in a plug 26 for connection to an A/C power source. Indeed, the battery 22 may be rechargeable from the A/C source to enable the nebuliser 16 to be used at sites remote from A/C power sources. A solar power system may also be used to drive the compressor 18, as may a DC 12V power source.

It is preferable that the medicament is supplied to the point of use in dry powder form, e.g. in a suitable phial. The dry powder medicament is then reconstituted at the point of use by a suitably trained person by adding a liquid dilutant of e.g. sterile water to produce a suitable volume of fluid containing the medicament.

The liquid medicament is then placed inside a pot of the nebuliser 16, and the nebuliser 16 is then driven by the compressor 18 to supply the vaccine to the interior of the bag 14. To do this the bag 14 is placed over the outlet 28 of the nebuliser 16 to cause the medicament, in aerosol form, to enter the hollow interior 12 of the bag 14. The compressor 18 may be operable to drive the nebuliser 16 for a measured period of time, which period of time is chosen to provide a suitable amount of aerosol into the bag 14. 30 seconds may be a suitable time.

To prevent escape of the aerosol from the bag 14 between the time in which the aerosol is supplied from the nebuliser 16 and the time in which the opening 30 of the bag is placed over the mouth and nose of the patient 10, the bag 14 needs to be closed. In practice, it is found that manual constriction of the bag 14 achieves sufficient sealing that there will be sufficient aerosol in the bag 14, assuming that the bag 14 is promptly transferred from the nebuliser 16 to the patient 10. To assist in this, the bag 14 preferably has a constriction 32 forming a neck, which can be clasped between the first and second fingers of the person transferring the bag 14 from the nebuliser 16 to the patient 10 (which may not be the patient themselves, particularly if the patient is a child). The constriction 32 is constricted over the outlet 28 of the nebuliser when the aerosol is supplied to the interior 12 of the bag 14, and then further constricted during transfer from the nebuliser 16 to the patient 10. Whilst more elaborate sealing arrangements are possible, they will make the bag more expensive to produce.

The pot of the nebuliser 16 is preferably disposable so that again there is no cross-contamination by e.g. re-filling the pot.

The medicament is preferably a vaccine, more preferably a measles vaccine.

Once the bag 14 has been placed over the nose and mouth of the patient 10, the patient breathes in and out of the bag for e.g. 30 seconds, to inhale the aerosol. The bag will inflate and deflate whilst the patient 10 is breathing, thereby giving a visible indication that the patient is indeed inhaling the aerosol. This is particularly useful when the patient is a baby or child, who may not be able to give a verbal account of their behaviour.

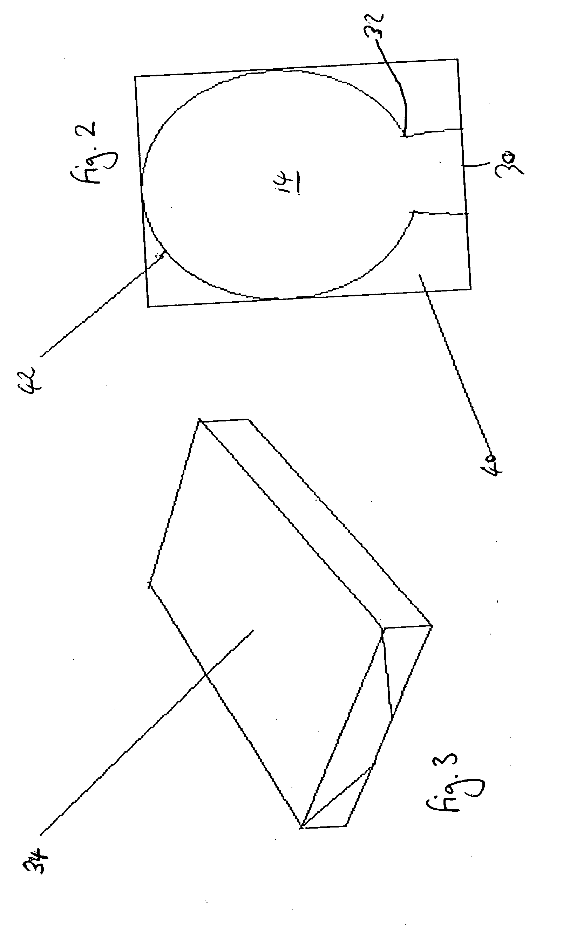

Since the amount of aerosol required by a single patient is small, a suitable sized nebuliser pot will provide sufficient medicament for a large number of patients (40 or so). Thus, it is desirable for the bag 14 for each patient to be delivered to the treatment site in convenient form. Thus, for example, as shown in Fig.2, the bag may be formed by a two-ply sheet 40 of edible material (e.g. rice paper), which sheet has a perforation 42 therein defining the periphery of the bag. The plies of the two-ply sheet 40 are sealed together immediately inside the perforation 42, so that the two plies form respective walls of the bag 14 when the bag 14 is released from the sheet 40. To release the bag 14, the sheet 40 is torn along the perforation 42, and the parts of the sheet 40 which do not correspond to the bag 14 can then be disposed of by eating.

Fig.2 also shows clearly the constriction 32 forming the neck of the bag 14, and how the bag splays between the constriction 32 and the opening 30.

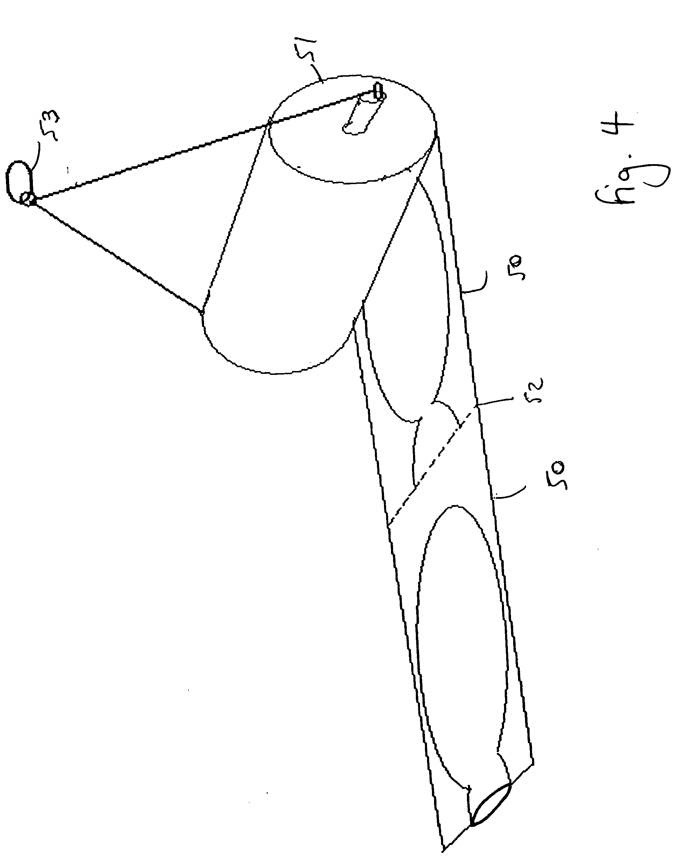

The sheet illustrated in Fig.2 may be provided in a block 34 of such sheets, as illustrated in Fig.3. Alternatively, a plurality of sheets, corresponding to sheet 40 shown in Fig.2, may be supplied end-to-end, with perforations between each sheet, so that a sheet to be used can be torn off from the other sheets. Such end-to-end connection of sheets could enable the sheets to be provided in a roll, rather than in the block 34 of Fig.3.

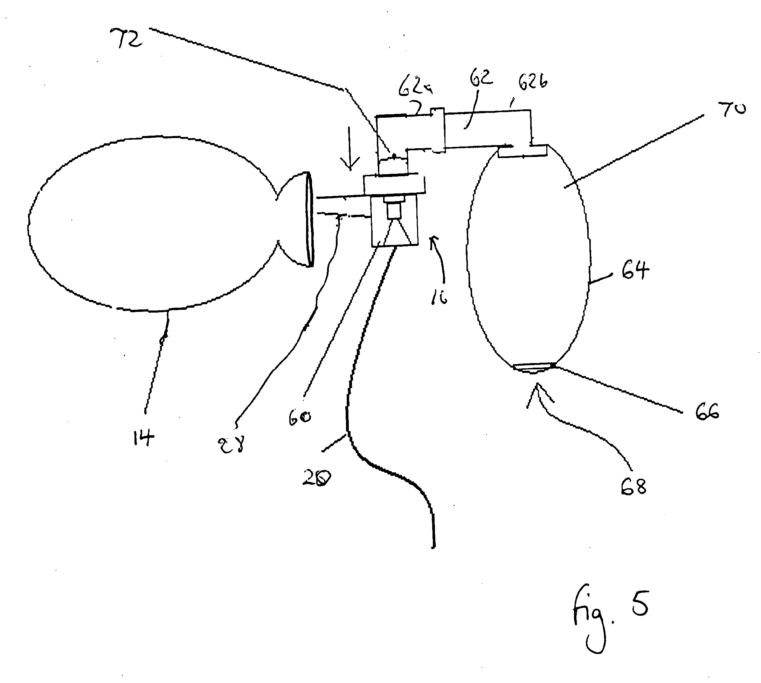

This arrangement is illustrated in Fig. 4.

Fig. 4 shows that plurality of sheets 50, which may be identical to the sheets 40 shown in Fig. 2, may be attached end to end on a roll 51. The sheets are separated by perforations 52. The roll may be suspended, as illustrated at 53.

Whether the sheets are provided in a roll so as in Fig. 4, or in a block 34 as illustrated in Fig.3, it is preferable that they are supplied to the treatment site enclosed within an anti-contamination protection.

One potential problem with the embodiment of Fig. 1 is that the nebuliser 16 needs to have sufficient power to inflate the bag 14. If it does not, the bag 14 will not be correctly filled with aerosol medicament. To overcome this problem, the arrangement of Fig. 1 may be modified as shown in Fig. 5. In Fig. 5, parts which correspond to those in Fig. 1 are indicated by the same reference numerals. Note also that Fig. 5 shows the nebuliser 16 in more detail, more clearly illustrating the pot 60 of the nebuliser. Note also that the compressor 18, power line 24 and plug 26 are omitted in Fig. 5.

In the embodiment illustrated in Fig. 5, the nebuliser 16 is connected via a duct 62 to reservoir 64. The reservoir 64 terminates, at its end remote from the duct 62, in a one-way valve 66. Thus, air can be introduced in the direction shown by arrow 68 into the hollow interior 70 of the reservoir 64. In use, the reservoir 64 may be squeezed at the time of activation of the nebuliser 16, to provide an airflow from the reservoir 64 through the duct 62 to the nebuliser 16, then through the outlet 28 to the bag 14. That airflow assists in the inflation of the bag 14. To prevent passage of aerosol to the reservoir 64, a further one-way valve 72 may be provided in the duct 62. It is convenient if the duct 62 is formed to L-shaped parts 62a, 62b as shown in Fig. 5, so that both the nebuliser 16 and the reservoir 64 can be below the duct 62. The components of the duct 62 may be disposed of at the same time as the disposal of the nebuliser 16, again reducing the risk of cross-infection.

As will be appreciated from the above, the components needed for treatment of patients are simple, and thus may be provided at low cost. This is particularly of value where the invention is to be used in less developed countries. The bag is simple to produce in bulk and the edible nature of the bag provides a ready, and acceptable method of disposal. Indeed, the impregnation of the bag with vitamins and/or nutritional minerals and/or with a flavouring which encourages the patient (or more probably the parent or other carer of the patient) to allow the patient to consume the bag rather than putting the bag to some other use. That way, bags are unlikely to be used to supply medicament to multiple patients, thereby reducing the risk of cross-contamination or environmental waste.

## Claims

1. A kit of parts for use in delivery or a medicament to a patient, comprising:
at least one bag of flexible material,
a container for said medicament; and
means for generating an aerosol of at least part of said medicament in said container for supply of said aerosol to the interior of the bag.

2. A kit of parts according to claim 1, wherein the bag is of edible material

3. A kit of parts according to claim 1, wherein the bag is of a food-derived polymer.

4. A kit of parts according to any one of the preceding claims, wherein the bag is part of a sheet, the sheet being perforated to permit separation of the bag from the rest of the sheet.

5. A kit of parts according to claim 4, wherein said sheet is one of a plurality of identical sheets.

6. A kit of parts according to any one of the preceding claims, wherein the bag is impregnated with vitamins and/or flavouring and/or nutritional minerals.

7. A kit of parts according to any one of the preceding claims, wherein the bag has a constricted neck, and is splayed between said neck and an opening for fitting over the nose and/or mouth of said patient.

8. A kit of parts according to any one of the preceding claims wherein said means for generating an aerosol is a nebuliser.

9. A kit of parts according to claim 8, further including a compressor for driving said nebuliser.

10. A medicament delivery device comprising a bag of flexible material, and a medicament in aerosol form in said bag, wherein said bag is closed but is openable to fit over the nose and/or mouth of a patient.

11. A medicament delivery device according to claim 10, wherein the bag is of edible material.

12. A medicament delivery device according to claim 10 or 11, wherein the bag is of rice paper.

13. A medicament delivery device according to claim 10 or 11, wherein the bag is of a food-derived polymer.

14. A medicament delivery device according to any one of claims 10 to 13, wherein the bag is impregnated with vitamins and/or flavouring and/or nutritional minerals.

15. A medicament delivery device according to any one of claims 10 to 14, wherein the bag has a constricted neck, and is splayed between said neck and an opening for fitting over the nose and/or mouth of said patient.

16. A bag for use in a method of delivery of a medicament in aerosol form to a patient, the bag having an opening for fitting over the nose and/or mouth of a patient.

17. A bag according to claim 16 of edible material.

18. A bag according to claim 16 or 17, wherein the bag is of rice paper.

19. A bag according to claim 16 or 17, wherein the bag is of a food-derived polymer.

20. A bag according to any one of claims 16 to 19, wherein the bag is part of a sheet of edible material, the sheet being perforated to permit separation of the bag from the rest of the sheet.

21. A bag according to claim 20, wherein said sheet is one of a plurality of identical sheets.

22. A bag according to any one of claims 16 to 21, wherein the bag is impregnated with vitamins and/or flavouring and/or nutritional minerals.

23. A bag according to any one of claims 16 to 22, wherein the bag has a constricted neck, and is splayed between said neck and said opening.

24. Use of a bag for the production of a medicament delivery device for treatment of a vaccinable disease by supply of said medicament in aerosol form for inhalation by a patient, the bag having an opening for fitting over the nose and/or mouth of a patient.

25. A use according to claim 24, wherein the bag is of edible material.

26. A use according to claim 24 or 25, wherein the bag is of rice paper.

27. A use according to claim 24 or 25, wherein the bag is of a food-derived polymer.

28. A use according to any one of claims 24 to 27, wherein the bag is part of a sheet of edible material, the sheet being perforated to permit separation of the bag from the rest of the sheet.

29. A use according to claim 28, wherein said sheet is one of a plurality of identical sheets.

30. A use according to any one of claims 24 to 29, wherein the bag is impregnated with vitamins and/or flavouring and/or nutritional minerals.

31. A use according to any one of claims 24 to 30, wherein the bag has a constricted neck, and is splayed between said neck and said opening.
